# EUROPÄISCHE PATENTSCHRIFT

(11) **EP 0 330 051 B1**
(45) Veröffentlichungstag und Bekanntmachung des Hinweises auf die Patenterteilung: **03.01.1996**
(21) Anmeldenummer: 89102503.3
(22) Anmeldetag: 14.02.1989
(51) Int. Cl.: C12N 15/38

(54) **Strukturelles Phosphoprotein (pp28) des menschlichen Cytomegalovirus (HCMV), seine Herstellung und Verwendung**
Human cytomegalovirus structural phosphoprotein (pp28), its preparation and use
Phosphoprotéine structurelle du cytomégalovirus humain, sa préparation et son utilisation

(30) Priorität: 24.02.1988 DE 3805717
(43) Veröffentlichungstag der Anmeldung: 30.08.1989
(73) Patentinhaber: BEHRINGWERKE Aktiengesellschaft, D-35001 Marburg (DE)
(72) Erfinder: Mach, Michael, Dr., D-8520 Erlangen (DE); Meyer, Heidi, D-8521 Spardorf (DE); Bröker, Michael, Dr., D-3550 Marburg (DE); Lauffer, Leander, Dr., Boston, MA 02108 (US)

(56) Entgegenhaltungen:
- EP-A- 0 252 531
- EP-A- 0 268 014
- JOURNAL OF VIROLOGY, Band 61, Nr. 5, Mai 1987, Seiten 1358-1367, American Society for Microbiology; G. JAHN et al.: "Map position and nucleotide sequence of the gene for the large structural phosphoprotein of human cytomegalovirus"
- VIROLOGY, Band 132, 1984, Seiten 325-338, Academic Press, Inc.; B. NOWAK et al. : "Characterization of monoclonal antibodies and polyclonal immune sera directed against human cytomegalovirus virion proteins"
- JOURNAL OF VIROLOGY, Band 59, Nr. 3, September 1986, Seiten 714-727, American Society for Microbiology; C. ROBY et al.: "Characterization of phosphoproteins and protein kinase activity of virions, noninfectious enveloped particles, and dense bodies of human cytomegalovirus"
- JOURNAL OF VIROLOGY, Band 62, Nr. 7, July 1988, Seiten 2243-2250, American Society for Microbiology; H. MEYER et al.: "Identification and procaryotic expression of the gene coding for the highly immunogenic 28-kilodalton structural phosphoprotein (pp28) of human cytomegalovirus"
- Virology 132, 1984, 325-338 ("Dokc")

## Beschreibung

Die Erfindung betrifft ein strukturelles HCMV-Phosphoprotein von 28 kD (pp28) und immunogene Teile davon, seine gentechnische Herstellung sowie seine Verwendung als Diagnostikum und zur Erzeugung von Antikörpern und Impfstoffen.

In mehreren Untersuchungen sind HCMV-Polypeptide mit Molekulargewichten von etwa 28 kD bisher beschrieben worden. Roby und Gibson (Roby, C. und Gibson, W. (1986) J. Virol. 59, 714-727) und Nowak et al. (Nowak, B. et al. (1984) Virology 132, 325-338) beschreiben ein Phosphoprotein von etwa 24 kD bzw. 29 kD. In beiden Veröffentlichungen wird vermutet, daß dieses Protein in der Matrix lokalisiert ist. Pereira et al. (Pereira, L. et al. (1984) Virology 139, 73-86) beschreiben einen monoklonalen Antikörper, der aus infizierten Zellextrakten ein 25 kD Glykoprotein präzipitiert, das zum Glykoprotein D-Komplex gehört. Irmiere und Gibson (Irmiere, A. und Gibson, W. (1985) J. Virol. 56, 277-283) haben ein 28 kD Protein identifiziert, das sowohl im A- als auch im B-Kapsid verschiedener HCMV-Stämme gefunden wurde. Re et al. (Re, M.C. et al. (1985) J. Gen. Virol. 66, 2507-2511) untersuchen ein 28 kD Strukturprotein mit Hilfe eines monoklonalen Antikörpers P2G11 (MAK P2G11). Dabei bleibt offen, ob es sich um das von Pereira et al. (a.a.O.) oder um das von Nowak et al. (a.a.O.) beschriebene Protein handelt.

Da ein Strukturprotein von 28 kD von fast allen hochpositiven humanen Seren erkannt wird, und somit unter den 28 kD-Proteinen eines der Hauptimmunogene des HCMV zu finden ist, erschien es wünschenswert, das dafür kodierende HCMV-Gen zu identifizieren und zu isolieren. Ein weiteres Ziel bestand darin, dieses Gen dann in geeigneten Wirtsystemen zu exprimieren, um es genauer zu charakterisieren und gegebenenfalls als weiteres Hauptimmunogen von HCMV zu etablieren.

Es wurde gefunden, daß mit dem MAK P2G11 (Re et al. a.a.O.) aus einer HCMV-cDNA-Genbank Klone identifiziert und isoliert werden können, die für ein 28 kD-Phosphoprotein (pp28) kodieren. Bisher war ein 28 kD großes Phosphoprotein nicht bekannt.

Zur Herstellung der Genbank wurde aus mit HCMV, Stamm Ad 169, infizierten menschlichen VorhautVorhaut-Fibroblastenzellen 96 bis 120 Stunden nach der Infektion die poly(A)⁺-RNA isoliert, in ds-DNA überführt und ohne Größenfraktionierung in den handelsüblichen Phagen-Expressionsvektor lambdagt11 eingefügt. Hierzu wurde der Vektor mit EcoRI gepalten und mit alkalischer Phosphatase (aus Kälberdarm) behandelt, um die intramolekulare Religation zu unterdrücken. Durch Anfügen von EcoRI-Linkern wurde die cDNA zwischen die Phagenarme eingefügt und in vitro verpackt. Aus 100 ng ds-cDNA wurde so eine Genbank erhalten, die etwa 5·10⁵ unabhängige Rekombinanten und 18% Wildtyp-Phagen enthielt.

Das "Screening" der Genbank erfolgte nach der Methode von R.A. Young und R.W. Davis, Proc. Natl. Acad. Sci. USA 80 1983) 1194-1198, jedoch mit der Abwandlung, daß Meerrettich-Peroxidase an Protein A gekoppelt wurde und 4-Chlor-1-naphthol als Detektionssystem diente, unter Einsatz des vorstehend beschriebenen monoklonalen Antikörpers P2G11. Bei diesem "Immunoscreening" werden die auf Nitrozellulosefiltern vorhandenen Kolonien vorsichtig inkubiert und nach Entfernen ungebundener Reaktanden positive Plaques mit dem genannten modifizierten Detektionssystem nachgewiesen.

Beim "Screening" der Genbank mit MAK P2G11 wurden von 150 000 rekombinanten lambdagt11 Phagen zwei positive Signale erhalten. Ein Klon mit einer Insertion von etwa 270 Basenpaaren (bp) wurde für die weitere Charakterisierung ausgewählt und gereinigt; er erhielt die Bezeichnung BUML-1.

Der E.coli Stamm Y 1089 wurde mit dem rekombinierten Phagen infiziert und die Synthese des β-Galaktosidase-Fusionsproteins durch Zugabe von Isopropylthiogalaktosid (IPTG) induziert. Hierbei wurde ein Fusionsprotein von etwa 130 kD gebildet, das deutlich größer als die β-Galaktosidase (118 kD) ist und das nicht in E.coli-Zellen gefunden wird, die mit lambdagt11 infiziert sind; es ist auch nicht in mit BUML-1 infizierten, aber nicht induzierten Zellen vorhanden. In "Western Blot" Analysen reagiert nur das 130 kD Polypeptid mit MAK P2G11. Es ergibt sich somit, daß der rekombinierte Klon BUML-1 ein Fusionsprotein mit einem HCMV-Protein-Anteil synthetisiert.

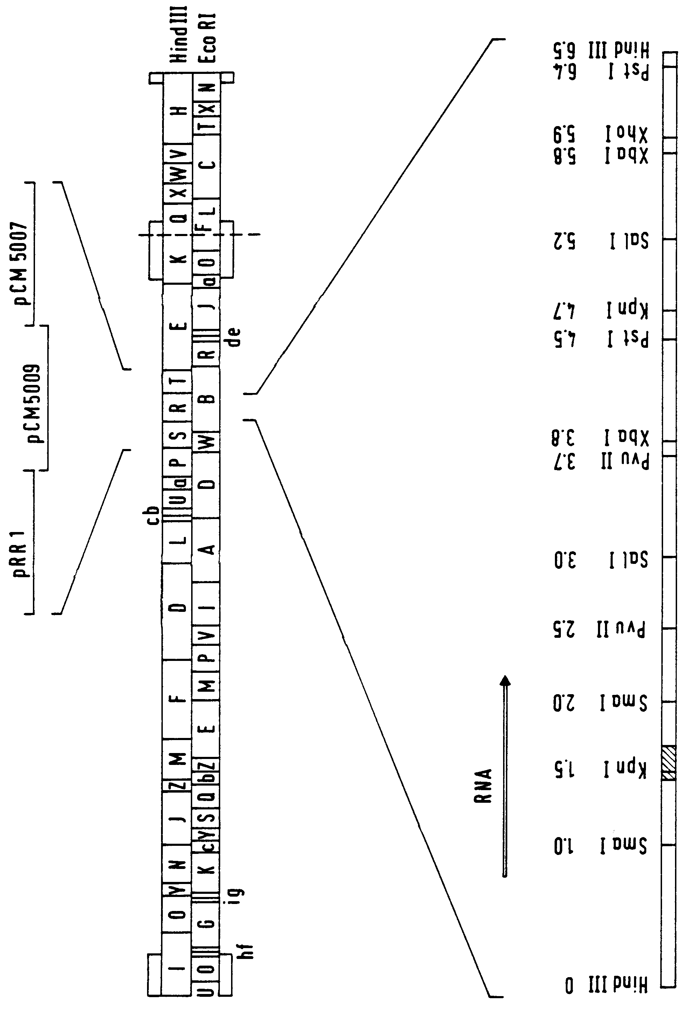

Die cDNA-Insertion von etwa 270 bp wurde nun verwendet, um das Gen für das HCMV-Protein im Virus-Genom zu lokalisieren: Hierfür wurde die o.g. cDNA-Insertion mit 8 Cosmid Klonen hybridisiert, die das gesamte Genom von HCMV umspannen (B. Fleckenstein et al. (1982) Gene 18, 39-46). Das Cosmid pCM 1058, das die HindIII-Fragmente P, R und S enthält, hybridisierte mit der cDNA. Eine eingehendere "Southern Blot"-Analyse dieser Region begrenzte das HCMV-DNA-Fragment auf ein 500 bp KpnI/SmaI-Fragment am linken Ende des HindIII-R-Fragments (Fig. 1). Mittels einer SstII-Spaltstelle war es möglich, die cDNA dem rechten KpnI/SmaI-Fragment in der Genomorientierung wie in Fig. 1 gezeigt zuzuordnen.

In "Northern Blot"-Analysen hybridisierte das cDNA-Fragment von BUML-1 am stärksten zu einer 1,3 kb großen späten mRNA, die vollständig vom HindIII-R-Fragment her transkribiert wird.

10 von 14 zufällig gewählten HCMV-positiven Seren reagierten mit p28, das aus gereinigten Viren stammte; dieselben Seren reagierten ebenfalls mit einem Fusionsprotein (p271, siehe Beispiel 1) aus gentechnischer Synthese. Bei in vitro phosphorylierten HCMV-Partikeln (phosphoryliert analog zu Roley, C. und Gibson, W. a.a.O.) konnte ein phosphoryliertes p28 sowohl mit MAK P2G11 als auch mit Antikörpern gegen das Fusionsprotein p271 (siehe Beispiel 1) präzipitiert werden. Das spricht dafür, daß das klonierte Protein phosphoryliert ist und somit als pp28 bezeichnet werden kann.

Da pp28 in den Mengen, die für Diagnostika und Vakzinen erforderlich sind, nur unter großem technischen Aufwand zu isolieren wäre, ist die erfindungsgemäße gentechnische Herstellungsweise besonders vorteilhaft. Es zeigte sich, daß nicht nur von eukaryotischen Zellen exprimierte Produkte antigen wirken, sondern auch Expressionsprodukte von Bakterien. Da Bakterien von Fremdgenen keine Phosphoproteine erzeugen, war nicht zu erwarten, daß auch bakteriell hergestelltes "HCMV-pp28" oder Teile davon stark immunogen wirken. Es zeigte sich jedoch, daß auch solche Proteine von entsprechenden Seren ebenso eindeutig erkannt werden wie authentisches pp28.

Die Erfindung betrifft folglich
a) Isolierte DNA, bestehend aus dem 500 bp Kpnl-Smal-Fragment innerhalb des HindIII-R-Fragments aus dem Genom von HCMV, Stamm Ad 169, oder einem dazu äquivalenten Fragment eines anderen HCMV-Stammes, welches' Fragment mit dem 500 bp Kpnl-Smal-Fragment auf dem HindIII-R-Fragment unter stringenten Bedingungen hybridisiert und für immunogene Teile von pp28 von HCMV kodiert.
b) diese DNA ganz oder teilweise enthaltende Vektoren
c) mit solche Vektoren transformierte pro- oder eukaryotische Zellen,
d) die von diesen Zellen aufgrund der Transformation exprimierten Polypeptide oder Teile davon,
e) die Verwendung dieses DNA bzw. exprimierten Polypeptide oder Teile davon als Diagnostikum
f) Impfstoffe gegen HCMV, die Peptide von (e) alleine oder in Kombination oder Antikörper gegen diese Proteine oder Teile davon enthalten
h) sowie ein Verfahren zur gentechnischen Herstellung der unter (d) angeführten Polypeptide oder Teilen davon.

Weitere Ausgestaltungen der Erfindung sind in den nachfolgenden Beispielen und den Patentansprüchen definiert.

### Beispiel 1: Konstruktion und Expression des Plasmids p271

Zunächst wurde das Plasmid pHM 7 erzeugt durch Einbau des ca. 500 bp KpnI-SmaI Fragments in M13mp11. Man erhält das Plasmid p271 durch Insertion des ca. 500 bp EcoRI-SmaI-Fragments von pHM 7 in pEX-2 (Stanley, K. und P. Luzio (1984) EMBO Journal 3, 1429-1434). Insertion in pEX 1 und pEX 3 ergab keine Fusionsproteine. Das Plasmid p271 kodiert also für ein Fusionsprotein von etwa 133 kD, bestehend aus einem pp28 Anteil (ca. 18 kD) fusioniert mit einem Cro/β-Galaktosidase-Hybridprotein (ca. 115 kD). Die Induktion der Expression in geeigneten E.coli-Stämmen erfolgt durch Hitzeinaktivierung eines temperatursensitiven Repressors. E.coli pop 2136 (Stanley, K. und P. Luzio a.a.O.) wurde zu einer Dichte von 0,2-0,3 (A₆₀₀ₙₘ) bei 30°C angezüchtet und die Synthese des Fusionsproteins durch schnellen Temperaturwechsel auf 42°C induziert. Nach 90 Min. bei 42°C wurden die Zellen geerntet und das pp28 Protein nach bekannten Methoden gereinigt. Das Fusionsprotein beträgt etwa 5% der Gesamtproteinmasse.

### Beispiel 2: Expressionsoptimierung von pp28

Durch die nachstehend beschriebenen Umklonierungen konnte (1) die Expression von pp28 als Fusionsprotein insgesamt verbessert und (2) der Fremdanteil im Fusionsprotein verkleinert werden. Als Ausgangsplasmid wurde pBD2IC2OH (Europäische Patentanmeldung EP 0 236 978) gewählt. Dieses Plasmid kodiert also für einen gegenüber pEX-2 wesentlich (um mehr als 60%) verkürzten β-Galaktosidase-Anteil, die Induktion erfolgte über das Lac-Promoter-System durch Zugabe von Isopropylthiogalaktosid (IPTG).

Zwei Strategien wurden zur Klonierung des pp28-DNA-Fragments aus p271 gewählt:
1) p271 wurde mit EcoRI und SmaI geschnitten, die EcoRI-Überhänge mittels Klenow-Fragment aufgefüllt. Das resultierende ca.500 bp-Fragment wurde in die SmaI-Stelle von pBD2IC2OH eingesetzt. Die Linkerregion des erzeugten Plasmids pGB10 hat die folgende DNA-Sequenz (verifiziert durch indirektes Sequenzieren am Doppelstrang): 
(S) E (aufge-          S
(m) c  füllt)          m
(a) o                  a
(I) R                  I
    I
GATGGGGATCCCCAATTCGAGCTCGCCCGACC...CCCGGG
---------+---------+---------ca. 500bp---
CTACCCCTAGGGGTTAAGCTCGAGCGGGCATGG:::GGGCCC
aus pBD2IC20H>< aus p 271   >< pp28...   >
AspGlyAspProGlnPheGluLeuAlaArgThr...ProGly
2) Der Leserahmen in der Linkerregion von pBD2IC2OH wurde durch Auffüllen der Bam HI-Stelle verschoben (--> Plasmid pGB11). Danach konnte das Eco RI/SmaI-Fragment aus p271 ohne vorheriges Auffüllen der Eco RI-Stelle direkt in das mit Eco RI und NruI geschnittene pGB11 eingesetzt werden. Die Linkerregion des resultierenden Plasmids pGB12 hat die folgende DNA-Sequenz:

E                                    (S N)
c                                    (m r)
o                                    (a u)
R                                    (I I)
I
GATGGGGATCGATCCCCGGGTACCGAGCTCGAATTCGAGCTCGCCCGTACC...CCCCGA
---------+---------+---------+---------+-------ca. 500bp----
CTACCCCTAGCTAGGGGCCCATGGCTCGAGCTTAAGCTCGAGCGGGCATGG...GGGGCT
aus pGB11                    >< aus p271    >< pp28... >
AspGlyAspArgSerProGlyThrGluLeuGluPheGluLeuAlaArgThr...ProArg

Die Abschätzung der Färbeintensität der entsprechenden Proteinbanden nach Auftrennung von E. coli-Gesantextrakten ergab, daß nach Induktion des p271-kodierten Proteins dieses etwa 5% des Gesamtproteingehalts ausmachte, während die entsprechenden Werte für pGB10 und pGB12 durchgängig bei etwa 20% lagen. Wird der verringerte Fremdproteingehalt in den pGB10-und pGB12-kodierten gegenüber dem p271-kodierten Fusionsprotein in Betracht gezogen, ergibt sich durch die Verwendung von pGB10 bzw. pGB12 eine etwa zehnfache Steigerung der pp28-Expression.

### Legende zu Fig. 1

Oberer Teil: Physikalische Genomkarte von HCMV für die Restriktionsendonukleasen HindIII und EcoRI.

Unterer Teil: Restriktionskarte für das HindIII-R-Fragment. Die schraffierte Region zeigt den Bereich der cDNA des Klons BUML-1.

## Patentansprüche (Patentansprüche für folgende(n) Vertragsstaat(en): AT, BE, CH, DE, FR, GB, IT, LI, LU, NL, SE)

1. Isoliertes strukturelles Phosphoprotein mit einem Molekulargewicht von etwa 28 kD (pp28) aus humanem Cytomegalovirus (HCMV) oder immunogene Teile davon, erhältlich durch Expression des Gens, das im Bereich des Fragments Hindlll-R des Genoms von HCMV, Stamm Ad 169 liegt oder durch Expression von für immunogene Bereiche von pp28 kodierenden Teilen dieses Gens.

2. Immunogener Teil von pp28, erhalten durch Expression des Gens, das auf einem 500 bp Kpnl-Smal-Fragment innerhalb des Hindlll-R-Fragments aus dem Genom von HCMC, Stamm Ad 169, liegt oder Teilen dieses Gens oder von dazu äquivalenten immunogenen Teilen von pp28 anderer HCMV-Stämme, wobei die für die äquivalenten Fragmente kodierenden DNA-Abschnitte mit dem 500 bp Kpnl-Smal-Fragment auf dem Hindlll-R-Fragment unter stringenten Bedingungen hybridisieren.

3. Isolierte DNA, bestehend aus dem 500 bp Kpnl-Smal-Fragment innerhalb des Hindlll-R-Fragments aus dem Genom von HCMV, Stamm Ad 169, oder einem dazu äquivalenten Fragment eines anderen HCMV-Stammes, welches Fragment mit dem 500 bp Kpnl-Smal-Fragment auf dem Hindlll-R-Fragment unter stringenten Bedingungen hybridisiert und für immunogene Teile von pp28 von HCMV kodiert.

4. Verfahren zur Herstellung eines strukturellen Phosphoproteins mit einem Molekulargewicht von etwa 28 kD (pp28) oder immunogenen Teilen davon aus humanem Cytomegalovirus (HCMV), dadurch gekennzeichnet, daß das Gen, welches im Bereich des Fragments Hindlll-R des Genoms von HCMV, Stamm Ad 169 liegt oder Teile dieses Gens, welche für immunogene Bereiche von pp28 kodieren, zur Expression gebracht werden.

5. Verfahren nach Anspruch 4, dadurch gekennzeichnet, daß das 500 bp Kpnl-Smal-Fragment innerhalb des HindIII-R-Fragments aus dem Genom von HCMV, Stamm Ad 169 eingesetzt wird, oder daß die äquivalenten DNA-Fragmente anderer HCMV-Stämme eingesetzt werden, wobei die äquivalenten DNA-Fragmente mit dem 500 bp Kpnl-Smal-Fragment auf dem Hindlll-R-Fragment unter stringenten Bedingungen hybridisieren.

6. Diagnostika, die Proteine oder Teile davon nach Anspruch 1 oder 2 enthalten.

7. Diagnostika, die DNA-Sequenzen oder Teile davon nach Anspruch 3 enthalten.

8. Impfstoffe, die Proteine oder Teile davon nach Anspruch 1 oder 2 enthalten.

9. Impfstoffe, die Antikörper gegen Proteine nach Anspruch 1 oder 2 enthalten.

## Patentansprüche (Patentansprüche für folgende(n) Vertragsstaat(en): ES)

1. Verfahren zur Herstellung eines strukturellen Phosphoproteins mit einem Molekulargewicht von etwa 28 kD (pp28) oder immunogenen Teilen davon aus humanem Cytomegalovirus (HCMV), dadurch gekennzeichnet, daß das Gen, welches im Bereich des Fragments HindIII-R des Genoms von HCMV, Stamm Ad 169 liegt oder Teile dieses Gens, welche für immunogene Bereiche von pp28 kodieren, zur Expression gebracht werden.

2. Verfahren nach Anspruch 1, dadurch gekennzeichnet, daß das 500 bp Kpnl-Smal-Fragment innerhalb des Hindlll-R-Fragments aus dem Genom von HCMV, Stamm Ad 169 eingesetzt wird, oder daß die äquivalenten DNA-Fragmente anderer HCMV-Stämme eingesetzt werden, wobei die äquivalenten DNA-Fragmente mit dem 500 bp Kpnl-Smal-Fragment auf dem Hindlll-R-Fragment unter stringenten Bedingungen hybridisieren.

3. Verfahren nach Anspruch 1 oder 2, dadurch gekennzeichnet, daß Fusionsproteine erzeugt werden.

4. Verfahren nach Anspruch 1, 2 oder 3, dadurch gekennzeichnet, daß Fusionsproteine mit β-Galaktosidase erzeugt werden.

## Claims (Claims for the following Contracting State(s): AT, BE, CH, DE, FR, GB, IT, LI, LU, NL, SE)

1. An isolated structural phosphoprotein having a molecular weight of about 28 kD (pp28) from human cytomegalovirus (HCMV) or immunogenic parts thereof, obtainable by expression of the gene which is located in the region of the fragment HindIII R of the genome of HCMV, strain Ad 169, or by expression of parts of this gene which code for immunogenic regions of pp28.

2. An immunogenic part of pp28, obtained by expression of the gene which is located on a 500 bp KpnI-SmaI fragment within the HindIII R fragment from the genome of HCMV, strain Ad 169, or parts of this gene or of immunogenic parts, equivalent thereto, of pp28 of other HCMV strains, where the DNA sections coding for the equivalent fragments hybridize with the 500 bp KpnI-SmaI fragment on the HindIII R fragment under stringent conditions.

3. An isolated DNA consisting of the 500 bp KpnI-SmaI fragment within the HindIII R fragment from the genome of HCMV, strain Ad 169, or of a fragment, equivalent thereto, of another HCMV strain, which fragment hybridizes with the 500 bp KpnI-SmaI fragment on the HindIII R fragment under stringent conditions and codes for immunogenic parts of pp28 of HCMV.

4. A method for the preparation of a structural phosphoprotein having a molecular weight of about 28 kD (pp28) or immunogenic parts thereof from human cytomegalovirus (HCMV), which comprises bringing about the expression of the gene which is located in the region of the fragment HindIII R of the genome of HCMV, strain Ad 169, or parts of this gene which code for immunogenic regions of pp28.

5. The method as claimed in claim 4, wherein the 500 bp KpnI-SmaI fragment within the HindIII R fragment from the genome of HCMV, strain Ad 169, is used, or wherein the equivalent DNA fragments of other HCMV strains are used, where the equivalent DNA fragments hybridize with the 500 bp KpnI-SmaI fragment on the HindIII R fragment under stringent conditions.

6. A diagnostic aid which contains proteins or parts thereof as claimed in claim 1 or 2.

7. A diagnostic aid which contains DNA sequences or parts thereof as claimed in claim 3.

8. A vaccine which contains proteins or parts thereof as claimed in claim 1 or 2.

9. A vaccine which contains antibodies against proteins as claimed in claim 1 or 2.

## Claims (Claims for the following Contracting State(s): ES)

1. A method for the preparation of a structural phosphoprotein having a molecular weight of about 28 kD (pp28) or immunogenic parts thereof from human cytomegalovirus (HCMV), which comprises bringing about the expression of the gene which is located in the region of the fragment HindIII R of the genome of HCMV, strain Ad 169, or parts of this gene which code for immunogenic regions of pp28.

2. The method as claimed in claim 1, wherein the 500 bp KpnI-SmaI fragment within the HindIII R fragment from the genome of HCMV, strain Ad 169, is used, or wherein the equivalent DNA fragments of other HCMV strains are used, where the equivalent DNA fragments hybridize with the 500 bp KpnI-SmaI fragment on the HindIII R fragment under stringent conditions.

3. The process as claimed in claim 1 or 2, wherein fusion proteins are produced.

4. The process as claimed in claim 1, 2 or 3, wherein fusion proteins with β-galactosidase are produced.

## Revendications (Revendications pour l'(les) Etat(s) contractant(s) suivant(s): AT, BE, CH, DE, FR, GB, IT, LI, LU, NL, SE)

1. Phosphoprotéine structurelle isolée ayant une masse moléculaire d'environ 28 kD (pp28) dérivant de cytomegalovirus humain (HCMV) ou fragments immunogènes de celle-ci, pouvant être obtenus par expression du gène qui se trouve dans le domaine du fragment Hindlll-R du génome de HCMV, souche Ad 169 ou par l'expression des fragments de ce gène codant pour des domaines immunogènes de pp28.

2. Fragment immunogène de pp28, obtenu par expression du gène qui se trouve dans un fragment Kpnl-Smal de 500 bp à l'intérieur du fragment Hind III-R du génome de HCMC, souche Ad 169 ou de fragments de ce gène ou fragments immunogènes équivalents de pp28 d'autres souches de HCMV, les séquences d'ADN codant pour les fragments équivalents s'hybridant dans des conditions stringentes avec le fragment Kpnl-Smal de 500 bp dans le fragment Hind III-R.

3. ADN isolé constitué du fragment Kpnl-Smal de 500 bp àl'intérieur du fragment Hindlll-R du génome de HCMV, souche Ad 169, ou d'un fragment équivalent d'une autre souche d'HCMV, fragment qui s'hybride dans des conditions stringentes avec le fragment Kpnl-Smal de 500 bp dans le fragment Hindlll-R et qui code pour des fragments immunogènes de la pp28 de HCMV.

4. Procédé pour l'obtention d'une phosphoprotéine structurelle ayant une masse moléculaire d'environ 28 kD (pp28) ou de fragments immunogènes de celle-ci dérivant de cytomegalovirus humain (HCMV), caractérisé en ce que l'on fait exprimer le gène qui se trouve dans le domaine du fragment Hindlll-R du génome de HCMV, souche Ad 169 ou des fragments de ce gène qui codent pour des domaines immunogènes de pp28.

5. Procédé selon la revendication 4, caractérisé en ce que l'on utilise le fragment Kpnl-Smal de 500 bp à l'intérieur du fragment Hindlll-R du génome de HCMV, souche Ad 169 ou des fragments d'ADN équivalents d'autres souches de HCMV qui hybrident dans des conditions stringentes avec le fragment Kpnl-Smal de 500 bp dans le fragment Hindlll-R.

6. Agents de diagnostic qui contiennent des protéines ou des fragments selon la revendication 1 ou la revendication 2.

7. Agents de diagnostic qui contiennent des séquences d'ADN ou des fragments selon la revendication 3.

8. Vaccins qui contiennent des protéines ou des fragments selon la revendication 1 ou 2.

9. Vaccins qui contiennent des anticorps dirigés contre des protéines selon les revendications 1 ou 2.

## Revendications (Revendications pour l'(les) Etat(s) contractant(s) suivant(s): ES)

1. Procédé pour l'obtention d'une phosphoprotéine structurelle ayant une masse moléculaire d'environ 28 kD (pp28) ou de fragments immunogènes de celle-ci dérivant de cytomegalovirus humain (HCMV), caractérisé en ce que l'on fait exprimer le gène qui se trouve dans le domaine du fragment Hindlll-R du génome de HCMV, souche Ad 169 ou des fragments de ce gène qui codent pour des domaines immunogènes de pp28.

2. Procédé selon la revendication 1, caractérisé en ce que l'on utilise le fragment Kpnl-Smal de 500 bp à l'intérieur du fragment Hindlll-R du génome de HCMV, souche Ad 169 ou des fragments d'ADN équivalents d'autres souches de HCMV qui hybrident dans des conditions stringentes avec le fragment Kpnl-Smal de 500 bp dans le fragment Hindlll-R.

3. Procédé selon la revendication 1 ou 2, caractérisé en ce que des protéines de fusion sont obtenues.

4. Procédé selon les revendications 1, 2 ou 3, caractérisé en ce que des protéines de fusion sont obtenues avec de la β-galactosidase.
